# EUROPEAN PATENT APPLICATION

(11) **EP 0 743 015 A1**
(43) Date of publication of application: **20.11.1996**
(21) Application number: 96302474.0
(22) Date of filing: 09.04.1996
(51) Int. Cl.: A23F 3/34, A61K 35/78, A23L 2/38

(54) **Health tea and method of producing same**

(30) Priority: 18.05.1995 JP 144063/95
(71) Applicant: Shimazakisyubyou Kabushiki Kaisya, Hamamatsu-shi, Shizuoka-ken (JP)
(72) Inventor: Shimazaki, Fujio, Hamamatsu-shi, Shizuoka-ken (JP)
(74) Representative: Jackson, Peter Arthur

(57) **Abstract**

There is disclosed a health tea comprising magnetized water containing ingredients of leaves of Gymnema Sylvestre. One method of producing the health tea comprises the steps of magnetizing water in a magnetic field, and dissolving ingredients of leaves of Gymnema Sylvestre into the magnetized water. Another method of producing the health tea comprises the steps of dissolving ingredients of leaves of Gymnema Sylvestre in water, and magnetizing this water, containing the dissolved ingredients, in a magnetic field.

## Description

This invention relates to a health tea for assisting heavy smoker to quit smoking, and drug (narcotic) addicts to quit narcotics.

It is said that the ratio of habitual smokers to the total population of Japan is 36.3% (The ratio for the men is 58.8% while the ratio for the women 15.2%) at present. Although this ratio is now decreasing, it is the case that there are still many habitual smokers in Japan.

Tobacco is deleterious to health, and thus antismoking campaigns have been extensively conducted. The number of smokers in the developed countries in the world is decreasing year by year.

In such an environment, there are still people who can not completely abstain from smoking even with the aid of smoke-abstaining tea, smoke-abstaining gum and the like. The reason why these people can not quit smoking is that when the concentration of nicotine in the blood goes below a predetermined level, their body craves nicotine. This is a kind of nicotine poisoning (addiction).

On the other hand, there is narcotism which can be a more serious social problem than nicotinism. Narcotic addicts are subjected to withdrawal distress when the concentration of narcotics ingredients in the blood goes below a predetermined level, and crave narcotics.

Whether or not addicts can be released from nicotinism or narcotism depends on how they can overcome the withdrawal distress when the concentration of its ingredients in the blood goes below the predetermined level. Namely, once they overcome such withdrawal distress, the concentration of the ingredients in the blood becomes close to that of normal people, and thereafter they no longer desire tobacco or narcotics.

With the above problems in view, it is an object of this invention to provide a health tea which lowers the concentration of nicotine or the like in the blood, and shortens a time period of withdrawal distress or eliminates it, thereby assisting addicts in quitting nicotinism or the like.

According to one aspect of the present invention, there is provided health tea comprising magnetized water containing ingredients of leaves of Gymnema Sylvestre.

According to another aspect of the invention, there is provided a method of producing health tea comprising a step of magnetizing water in a magnetic field; and a step of dissolving ingredients of leaves of Gymnema Sylvestre into the magnetized water.

According to a further aspect of the invention, there is provided a method of producing health tea comprising a step of dissolving ingredients of leaves of Gymnema Sylvestre into water; and a step of magnetizing the water, containing the dissolved ingredients, in a magnetic field.

In the present invention, since Gymnema Sylvestre causes smokers to experience a bitter taste, it assists in suppress a desire to smoke.

It is known that magnetized water exerts a certain influence on the permeability of a membrane, and activates the exchange of matters in a living system. As a result, magnetized water serves to discharge toxic substances from the body, thereby enhancing the metabolism. For example, it is reported that after magnetized water was drunk for one month and a half, cholesterol content in the blood serum was reduced from 67 mg% to 32 mg%. Thus, magnetized water enhances the metabolism, and enables toxic substances (e.g. nicotine) in the blood to be discharged the body.

The present invention will now be described specifically. Gymnema Sylvestre, used in the present invention, is a vine of Indian origin, and chewing leaves, suppresses the sence of sweetness on the palate. Therefore, in ancient times in India, this plant was called "gur-mar" or sugar destroyer. Gymnema Sylvestre is still used by people to promote beauty and health.

Gymnema Sylvestre is usually taken in the form of tea. When drinking Gymnema Sylvestre tea, its bitter taste, suppresses cravings for nicotine.

In the present invention, the ingredients of Gymnema Sylvestre are dissolved in magnetized water. The magnetized water is obtained by placing water in a strong magnetic field. Electromagnetic processing devices used for this purpose are classified into a type having a permanent magnet and a type having an electromagnet. The device with the permanent magnet has the advantage that it is simple in construction, and does not require electric wiring, but has a disadvantage that the intensity of the magnetic field can not be adjusted. On the other hand, the device with the electromagnet has features opposite to those of the device with the permanent magnet. Either of the two types can be used in the present invention.

Literature regarding the relation between magnetism and the living body is very limited at present, and the number of researchers in this field is very limited, and therefore this relation has not yet been fully clarified. However, it is known that there is close relation between the living body and electricity, and electricity always exists where magnetism exists, and therefore it is clear that magnetism has some influence on the body.

More specifically, when an electric field acts on a moving electric conductor, electromagnetic induction occurs, and this promotes the dissociation of electrolytes. For example, let's consider blood flow in a blood vessel. If a magnetic field acts on this blood flow in intersecting relation thereto, an electromotive force is produced in a direction perpendicular to both a component of the magnetic field perpendicular to the blood flow and the blood flow. This principle is the same as that obtained in an electromagnetic flow meter. It is thought that particles (ions) having a positive charge or negative charge in the blood are subjected to Lorentz's force in the magnetic field, and move in opposite directions, respectively, so that this electromotive force is produced.

In the present invention, when a toxis substance such as nicotine is placed in a magnetic field, hydration of ions of the toxic substance decreases, so that the permeability of the ions to a living body membrane is enhanced. As a result, it is thought that the toxic substance can be more easily discharged from the body. The present invention is directed to a kind of tea, and by taking this tea, a diuretic effect and sweating (perspiring) effect are achieved, and the effect of discharging a toxic substance such as nicotine through sweat and urine is promoted.

An electromagnet of 500 gauss was caused to act on water of 2 liters for 15 minutes, and the ingredients of dried Gymnema Sylvestre leaves of 6 grams were dissolved into this water (magnetized water), thereby preparing Gymnema Sylvestre tea. The inventor of the present invention and other habitual smokers drank this tea every day for three months, and were caused to sweat every day for a predetermined period of time. Everyone who participated in this test was able to quit smoking without undergoing withdrawal distress.

After taking coffee, sweets, liquors or the like, smokers tend to have a greater desire for smoking. However, if habitually taking the magnetized water containing the ingredients of Gymnema Sylvestre leaves, the desire for smoking is suppressed even when taking liquors or the like, since Gymnema Sylvestre causes a bitter taste. And besides, since the amount of the magnetized humor for causing the toxic substances (such as nicotine) to be rapidly discharged from the living body is increased, smoking can be easily quit.

Habitual smokers, who participated in a smoking-quitting experiment, took 2 liters of the health tea of the present invention every day, and took a bath for about one hour every day so as to promote sweating. Table 1 shows the relation between the number of cigarettes so far smoked a day by the participants and a time period required for quitting smoking.

**Table 1**

| Number of Cigarettes per Day (Brand: Seven Star) | Time Period required for quitting Smoking |
|---|---|
| About 20 | 1 month |
| About 40 | 2 months |
| About 60 | 3 months |
| About 80 | 4 months |
| About 100 | 56 months |

Next, two methods of producing the health tea of the present invention will be described below. The first production method comprises a step of placing water in a magnetic field to magnetize the water, and a step of dissolving the ingredients of Gymnema Sylvestre leaves into this magnetized water.

The water-magnetizing step can be effected by the use of the above-mentioned water-electromagnetizing devices, or by a method in which a coil or an electric wire is immersed in water so as to magnetize the water by a magnetic field produced by the coil or the electric wire. The water can be magnetized by any other known method.

The ingredients of Gymnema Sylvestre leaves can be dissolved in the water, for example, by a method in which these leaves are put in the magnetized water, and then the magnetized water is heated to be boiled, or by a method in which the magnetized water is boiled, and then the leaves of Gymnema Sylvestre are put into this water to dissolve its ingredients in the water. As another alternative, the ingredients are extracted from the leaves of Gymnema Sylvestre, and concentrated, and this concentrate solution may be mixed with magnetized water to prepare the health tea of the present invention. There can be used a method in which Gymnema Sylvestre leaves are powdered using a freeze-drying process or the like, and this powder is introduced into magnetized water. In these cases, the ingredients of Gymnema Sylvestre leaves are subsequently dissolved in the water.

The second production method of the present invention comprises a step of dissolving the ingredients of Gymnema Sylvestre leaves in water, and a step of magnetizing the water, containing the dissolved ingredients, in a magnetic field.

This second production method differs from the first production method in that the ingredients of Gymnema Sylvestre leaves are first dissolved in the water, but the method of dissolving the ingredients of Gymnema Sylvestre leaves in the water, as well as the method of magnetizing the water, containing the dissolved ingredients, in the magnetic field, is the same as that described in the first production method.

Therefore, by taking the health tea of the present invention, it is easy to quit smoking without withdrawal distress, since the concentration of nicotine in the blood quickly decreases.

Even if one can not completely quit smoking, the number of cigarettes smoked a day can be reduced. In this case, if such a person continues to drink the health tea of the present invention, he can continue to smoke a number of cigarettes without nicotinism, and can thus quit smoking of his own will.

In the case of narcotic addiction, by taking the health tea of the present invention, the concentration of narcotic ingredients in the blood is lowered to thereby alleviate withdrawal distress, and therefore narcotic addicts can relatively easily be released from narcotic addiction.

## Claims

1. A health tea comprising magnetized water containing ingredients of leaves of Gymnema Sylvestre.

2. A method of producing a health tea comprising:
a step of magnetizing water in a magnetic field; and
a step of dissolving ingredients of leaves of Gymnema Sylvestre into said magnetized water.

3. A method of producing a health tea comprising:
a step of dissolving ingredients of leaves of Gymnema Sylvestre into water; and
a step of magnetizing said water, containing said dissolved ingredients, in a magnetic field.
